# FASCICULE DE BREVET EUROPEEN

(11) **EP 2 331 555 B1**
(45) Date de publication et mention de la délivrance du brevet: **18.09.2013**
(21) Numéro de dépôt: 09772493.4
(22) Date de dépôt: 02.07.2009
(51) Int. Cl.: C07F 17/02, A61P 35/00, A61K 33/26

(54) **DÉRIVES FERROCENIQUES A ACTIVITE ANTICANCEREUSE**
Ferrocenderivate mit antineoplastischer Activität
FERROCENE DERIVATIVES HAVING ANTINEOPLASTIC ACTIVITY

(30) Priorité: 03.07.2008 FR 0854533
(43) Date de publication de la demande: 15.06.2011
(73) Titulaire: Centre National de la Recherche Scientifique (CNRS), 75016 Paris (FR)
(72) Inventeur: JAOUEN, Gérard, F-94240 L'Hay Les Roses (FR); VESSIERES-JAOUEN, Anne, F-94240 L'Hay Les Roses (FR); PLAZUK, Damian, PL-26-930 Garbatma Letnisko (PL)
(74) Mandataire: Regimbeau
(86) Numéro de dépôt international: PCT/EP2009/058309
(87) Numéro de publication internationale: WO 2010/000793

(56) Documents cités:
- HILLARD ET AL: "Organometallic diphenols: The importance of the organometallic moiety on the expression of a cytotoxic effect on breast cancer cells" JOURNAL OF ORGANOMETALLIC CHEMISTRY, ELSEVIER-SEQUOIA S.A. LAUSANNE, CH, vol. 692, no. 6, 12 février 2007 (2007-02-12), pages 1315-1326, XP005880405 ISSN: 0022-328X
- ABEYSINGHE P MANOHARI ET AL: "Antitumour bis(cyclopentadienyl) metal complexes: titanocene and molybdocene dichloride and derivatives." DALTON TRANSACTIONS (CAMBRIDGE, ENGLAND : 2003) 28 AUG 2007, no. 32, 28 août 2007 (2007-08-28), pages 3474-3482, XP002507577 ISSN: 1477-9226
- HILLARD E A ET AL: "The influence of phenolic hydroxy substitution on the electron transfer and anti-cancer properties of compounds based on the 2-ferrocenyl-1-phenyl-but-1- ene motif" DALTON TRANSACTIONS 2007 ROYAL SOCIETY OF CHEMISTRY; THOMAS GRAHAM HOUSE; SCIENCE PARK GB, no. 43, 2007, pages 5073-5081, XP002507578

## Description

La présente invention concerne des dérivés ferrocéniques utiles dans le traitement du cancer, ainsi que leur procédé de préparation et leurs utilisations.

L'intérêt de l'utilisation de dérivés de coordination métalliques en médecine a été ravivé par la découverte de Rosenberg des effets anticancéreux du cis-platine (Rosenberg, B. et al. Nature 1969, 222, 385-386 ; Rosenberg, B. et al. Nature 1965, 205, 698-699 ; Wong, E. et al. Chem. Rev. 1999, 99, 2451-2466). Il y a actuellement quatre représentants de ce type de complexes de coordination sur le marché. Ils figurent ci-dessous:

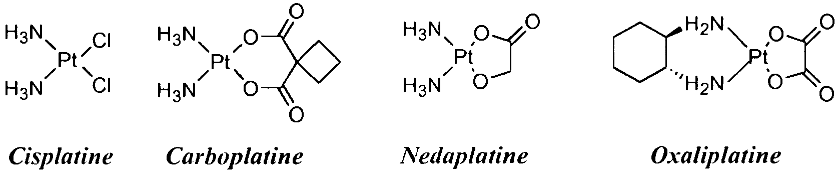

Après hydrolyse, ils agissent initialement par association directe à l'ADN de façon à prévenir la réplication cellulaire (Lippert, B. Cisplatin: Chemistry and Biochemistry of a Leading Anticancer Drug. John Wiley and Sons: New York, 1999.). Ces complexes souffrent cependant, malgré leur intérêt thérapeutique, de plusieurs faiblesses telles qu'une toxicité systémique sévère, et une gamme d'efficacité thérapeutique relativement étroite.

Il est cependant possible d'introduire de nouveaux paradigmes dans le domaine des métallodrogues en profitant de la versatilité de la chimie organométallique qui a permis l'ouverture d'une nouvelle interface (Vessières, A. et al. Dalton Trans. 2006, 4, 529-541 ; Jaouen, G. et al. Organometallics targeted to specific biological sites: The development of new therapies. In Bioorganometallics, Jaouen, G., Ed. Wiley-VCH: Weinheim, 2005; pp 65-95).

Ainsi, des composés ferrocéniques analogues structuraux de la chloroquine, médicament malheureusement résistant à de nouvelles souches de la malaria, ont fait preuve d'une activité antimalarique permettant de surmonter cette résistance au point que la ferroquine, l'un de ces composés ferrocéniques, est en phase clinique IIb chez Sanofi-Aventis. De même, deux complexes aréniques du ruthénium (A) et (B) sont en début d'essais cliniques comme antimétastasiques (Wand, F. et al. Inorg. Chem. 2002, 41, 4509-4523 ; Allardyce, C. S. et al. Chem. Commun. 2001, 1396-1397).

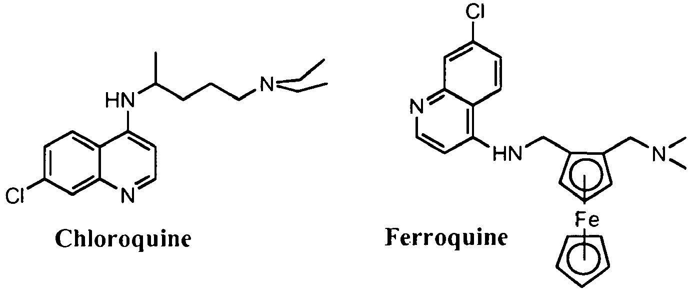

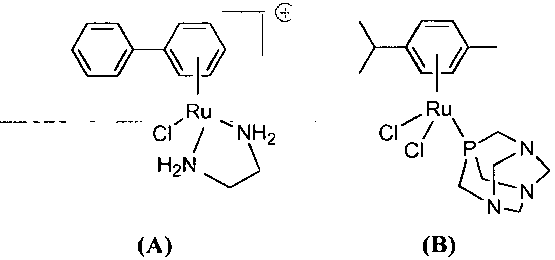

De même, les molécules 1, 2 et 3 ci-dessous ont été étudiées *in vitro* (Top, S. et al. Chem. Eur. J. 2003, 9, 5223-5236 ; Vessières, A. et al. J. Med. Chem. 2005, 48, 3937-3940 ; Top, S. et al. J. Organomet. Chem. 2001, 637, 500-506).

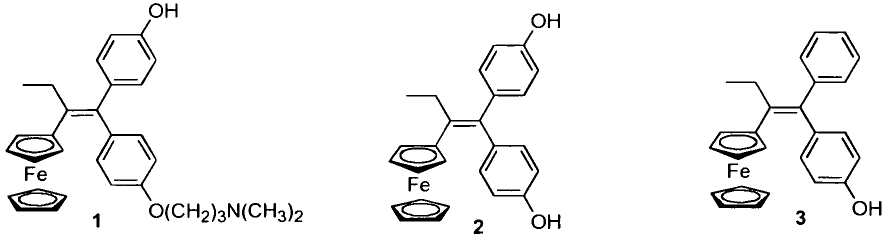

Ainsi, la molécule 1 ressemble pour partie dans ses effets au tamoxifène puisqu'elle présente le même type d'activité antioestrogénique sur des tumeurs du sein hormonodépendantes (classées ER+) mais s'en différencie par un comportement antiprolifératif sur des tumeurs non hormonodépendantes (ER-).

Les molécules 2 et 3, quant à elles, possèdent une activité antiproliférative sur des lignées de cancers du sein (MCF-7, MDA-MB-231) et de la prostate (PC-3, DU-145).

Malheureusement les molécules ouvertes ci-dessus 1, 2 et 3, ne sont pas encore optimales telles quelles pour un développement éventuel.

Les inventeurs ont ainsi découvert de manière surprenante une nouvelle famille de dérivés ferrocéniques ayant une activité anticancéreuse 10 à 20 fois supérieure à celle des molécules 1, 2 ou 3, notamment sur des lignées de cellules cancéreuses ne contenant pas de récepteur α des oestrogènes (correspondant à des cancers non hormono-dépendants).

La présente invention a donc pour objet un composé de formule (1) suivante : 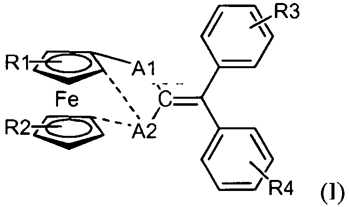 ou un sel pharmaceutiquement acceptable de celui-ci, un isomère ou un mélanges d'isomères en toutes proportions, en particulier un mélange d'énantiomères, et notamment un mélange racémique, ou un dérivé hydrosoluble, pour lequel :
- A1 et A2 représentent, indépendamment l'un de l'autre, soit une liaison entre le cyclopentadiényle et le carbone de la double liaison, soit une chaîne alkyle linéaire comprenant respectivement n1 et n2 atomes de carbone, éventuellement substituée par un ou plusieurs groupement(s) choisi(s) parmi un atome d'halogène ; un groupe phényle éventuellement substitué par un groupe OH ; un groupe (C₁-C₆)alkyle éventuellement substitué par un ou plusieurs atome(s) d'halogène ; et un groupe (C₃-C₆)cycloalkyle éventuellement substitué par un ou plusieurs atome(s) d'halogène,
   avec n1 et n2 représentant, indépendamment l'un de l'autre, un nombre entier compris entre 1 et 4,
   à la condition que A1 et A2 ne représentent pas simultanément une liaison et que la chaîne A1-C-A2 comporte au moins 3 atomes de carbone, et de préférence 3, 4 ou 5 atomes de carbone,
- ---- est absent ou représente une liaison, à la condition que A2 soit lié à un et un seul cylopentadiényle,
- R1 et R2 représentent chacun un atome d'hydrogène ou forment ensemble une chaîne alkyle linéaire reliant les deux cyclopentadiényles et comportant 3 à 5 atomes de carbone, cette chaîne étant éventuellement substituée par un ou plusieurs groupement(s) choisi(s) parmi un atome d'halogène ; un groupe phényle éventuellement substitué par un groupe OH; un groupe (C₁-C₆)alkyle éventuellement substitué par un ou plusieurs atome(s) d'halogène ; et un groupe (C₃-C₆)cycloalkyle éventuellement substitué par un ou plusieurs atome(s) d'halogène, et
- R3 et R4 représentent, indépendamment l'un de l'autre, un atome d'hydrogène ou un groupement CF₃, CN, OR⁵ ou NR⁶R⁷,
avec:
- R⁵ représentant un atome d'hydrogène ou un groupement -CO-(C₁-C₂₀)alkyle ou -(CH₂)ₘNR⁸R⁹,
- R⁶, R⁷, R⁸ et R⁹ représentant, indépendamment les uns des autres, un atome d'hydrogène ou un groupe (C₁-C₆)alkyle ou acyle, et
- m représentant un nombre entier compris entre 2 et 8.

Ainsi, les molécules de l'invention correspondent à la formule (Ia) ou (Ib) suivante : 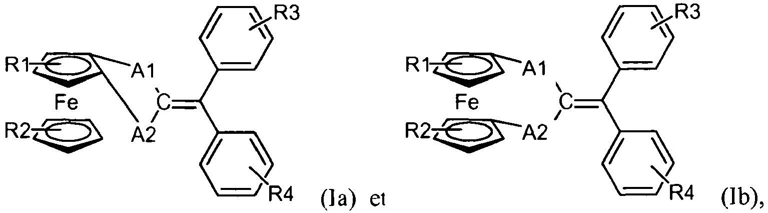 les groupements R1, R2, R3, R4, A1 1 et A2 étant tels que définis ci-dessus.

Avantageusement, les molécules de l'invention correspondront à la formule (Ib).

Par chaîne « A1-C-A2 », on entend la chaîne formée par les deux groupements A1 et A2 et l'atome de carbone de la double liaison lié à ces deux groupements.

Dans la présente invention, on entend désigner par « pharmaceutiquement acceptable » ce qui est utile dans la préparation d'une composition pharmaceutique, qui est généralement sûr, non toxique et ni biologiquement ni autrement non souhaitable et qui est acceptable pour une utilisation vétérinaire de même que pharmaceutique humaine.

On entend désigner par « sels pharmaceutiquement acceptables » d'un composé des sels qui sont pharmaceutiquement acceptables, comme définis ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphosulfonique, l'acide citrique, l'acide éthanesulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires. Avantageusement, il s'agit de l'acide chlorhydrique ; ou
(3) les sels formés lorsqu'un proton acide présent dans le composé parent soit est remplacé par un ion métallique, par exemple un ion de métal alcalin, un ion de métal alcalino-terreux ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium. Avantageusement, le proton acide est déplacé par un ion Na⁻, notamment en utilisant de l'hydroxyde de sodium.

Dans la présente invention, on entend désigner par « isomères », au sens de la présente invention, des diastéréoisomères ou des énantiomères. Il s'agit donc d'isomères de configuration encore appelés « stéréoisomères ». Les stéréoisomères qui ne sont pas des images dans un miroir l'un de l'autre sont ainsi désignés par « diastéréoisomères », et les stéréoisomères qui sont des images l'un de l'autre dans un.miroir mais non superposables sont désignés par « énantiomères », encore appelés « isomères optiques ».

Un atome de carbone lié à quatre substituants non identiques est appelé un « centre chiral ». Lorsqu'une molécule possède un tel centre chiral, elle est dite chirale et possède deux formes énantiomères. Lorsqu'une molécule possède plusieurs centres chiraux, alors elle possédera plusieurs formes diastéréoisomères et énantiomères.

Un mélange équimolaire de deux énantiomères est appelé mélange racémique.

Par « dérivé hydrosoluble », on entend désigner, au sens de la présente invention, des composés de formule (I) pour lesquels les groupements R3 et R4 sont tels qu'ils permettent d'augmenter la solubilité dans l'eau du composé et donc sa biodisponibilité. De tels composés seront en particulier des composés de formule (I) pour lesquels R3 et/ou R4 représente(nt) un groupement hydroxyle estérifié ou couplé à une espèce hydrosoluble, telle qu'un sucre ou un polymère hydrosoluble.

Par « sucre », on entend notamment, au sens de la présente invention, l'érythrose, le thréose, le ribose, l'arabinose, le xylose, le lyxose, l'allose, l'altrose, le glucose, le mannose, le gulose, l'idose, le galactose, le talose, l'érythrulose, le ribulose, le xylulose, le psicose, le fructose, le sorbose ou encore le tagatose, sous forme D ou L. Avantageusement, il s'agira du glucose ou du rhamnose.

Par « polymère hydrosoluble », on entend notamment au sens de la présente invention, un dendrimère ou un dérivé de polyéthylène glycol (PEG). Un dendrimère peut être en particulier un dendrimère de type polyamidoamide (PAMAM)

En particulier, le(s) groupement(s) R3 et/ou R4 peut(peuvent) représenter une chaîne dérivé de PEG de formule -COCH₂X¹CH₂COX²(CH₂CH₂O)_{P}CH₂CH₂X³, où :
- X¹ représente une liaison directe, un atome d'oxygène ou un groupement CH₂ ou CH₂-CH₂,
- X² représente un atome d'oxygène ou un groupement NH,
- X³ représente un groupement OR¹¹ ou NR¹²R¹³,
- R¹¹ représentant un atome d'hydrogène ou un groupement (C₁-C₆)alkyle,
- R¹² et R¹³ représentant, indépendamment l'un de l'autre, un atome d'hydrogène ou un groupe (C₁-C₆)alkyle,
- m représentant un nombre entier compris entre 2 et 8, et
- p représentant un nombre entier compris entre 1 et 20.

Par « chaîne alkyle », on entend au sens de la présente invention tout groupe hydrocarboné saturé, linéaire ou ramifié, sauf indication contraire.

Par groupe « (C₁-C₆)alkyle », on entend au sens de la présente invention un groupe alkyle tel que défini ci-dessus comportant de 1 à 6 atomes de carbone, et avantageusement de 1 à 4 atomes de carbone, en particulier les groupes méthyle, éthyle, n-propyle, isopropyle, *n*-butyle, *iso*-butyle, *sec*-butyle et *tert*-butyle.

Par groupe « (C₁-C₂₀)alkyle », on entend au sens de la présente invention un groupe alkyle tel que défini ci-dessus comportant de 1 à 20 atomes de carbone

Par groupe « (C₃-C₆)cycloalkyle », on entend au sens de la présente invention un cycle hydrocarboné saturé comportant de 3 à 6 atomes de carbone et encore avantageusement 3 ou 4 atomes de carbone, en particulier le groupe cyclopropyle ou cyclobutyle.

Par groupe « acyle », on entend au sens de la présente invention un groupe de formule -CO-R où R représente un groupe (C₁-C₆)alkyle tel que défini ci-dessus.

Par « halogène », on entend, au sens de la présente invention, un atome de fluor, de brome, de chlore ou d'iode. Avantageusement, il s'agira d'un fluor, d'un brome ou d'un chlore.

De manière avantageuse, au moins un des groupements R3 et R4 ne représente pas un atome d'hydrogène, et de manière encore plus avantageuse, les deux groupements R3 et R4 ne représentent pas un atome d'hydrogène.

Dans un mode de réalisation particulier, R3 et/ou R4 représentent, indépendamment l'un de l'autre, un groupement OR⁵, R⁵ étant tel que défini ci-dessus, et représentant de préférence un atome d'hydrogène.

De manière avantageuse, R3 et R4 représentent, indépendamment l'un de l'autre, un groupement OR⁵, R⁵ étant tel que défini ci-dessus, et représentant de préférence un atome d'hydrogène.

De manière encore avantageuse, R3 et R4 représentent un groupement OH.

Dans un autre mode de réalisation particulier, R3 et/ou R4 sont situés en position *para* sur le noyau phényle.

De manière avantageuse, R3 et R4 sont situés en position *para* sur le noyau phényle.

De manière encore avantageuse, R3 et R4 sont situés en position *para* sur le noyau phényle et représentent des groupements OH.

Dans un autre mode de réalisation particulier, R1 et R2 représentent chacun un atome d'hydrogène.

De manière avantageuse, A1 et A2 représentent des chaînes alkyles linéaires comportant respectivement n1 et n2 atomes de carbone, éventuellement substituées par un groupe (C1-C6)alkyle.

Selon un mode de réalisation avantageux, la chaîne A1-C-A2 comporte au plus 5 atomes de carbone. Ainsi, la chaîne A1-C-A2 comportera avantageusement 3, 4 ou 5 atomes de carbone, et avantageusement 3 atomes de carbone.

De manière encore avantageuse, A1 ou A2 représente une liaison.

Le composé selon l'invention pourra être choisi en particulier parmi les molécules de formules 4 à 11 suivantes :

| | | | |
|---|---|---|---|
| **4** | 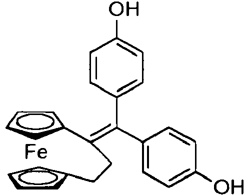 | **5** | 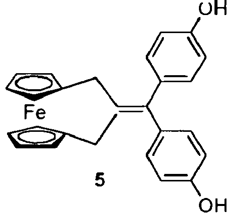 |
| **6** | 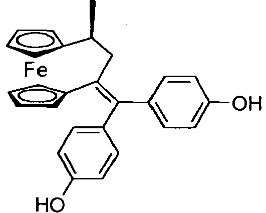 | **7** | 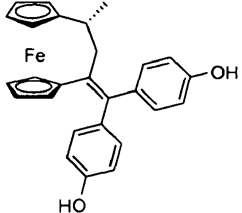 |
| **8** | 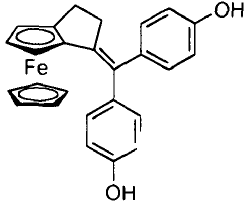 | **9** | 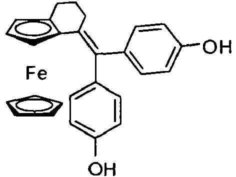 |
| **10** | 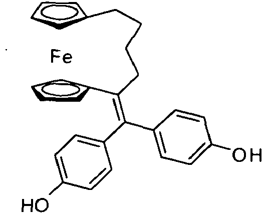 | **11** | 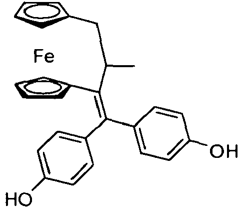 |
| **12** | 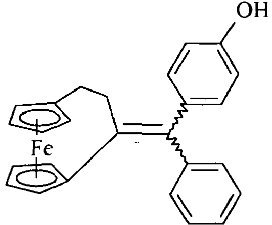 | | |

La présente invention a également pour objet les composés de formule (1) tels que définis ci-dessus pour leur utilisation en tant que médicament.

Ils pourront être utiles en particulier en tant que médicament antiprolifératif, notamment dans le traitement ou la prévention du cancer, et en particulier du cancer du sein et de la prostate. Encore plus avantageusement, les composés de l'invention seront utiles dans le traitement ou la prévention du cancer du sein, et de préférence hormono-indépendant.

La présente invention concerne également l'utilisation d'un composé de formule (1) tel que défini ci-dessus pour la fabrication d'un médicament, notamment destiné au traitement ou à la prévention de maladies prolifératives telles que le cancer, et en particulier, telles que le cancer du sein ou de la prostate. Encore plus avantageusement, les composés de l'invention seront utilisés dans le traitement ou la prévention du cancer du sein, et de préférence hormono-indépendant.

La présente invention concerne également une méthode de traitement ou de prévention d'une maladie proliférative, telle que le cancer, et en particulier le cancer du sein ou de la prostate, et avantageusement du cancer du sein, de préférence hormono-indépendant, comprenant l'administration d'une quantité suffisante d'un composé de formule (I) selon l'invention à un patient en ayant besoin.

La présente invention a également pour objet une composition pharmaceutique comprenant au moins un composé de formule (I) tel que défini ci-dessus, en association avec un véhicule pharmaceutiquement acceptable.

Cette composition pharmaceutique pourra comprendre au moins un autre principe actif, qui pourra être en particulier un composé anticancéreux avantageusement choisi parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide et le lanréotide.

Les composés selon l'invention peuvent être administrés par voie orale, sublinguale, parentérale, sous-cutanée, intramusculaire, intraveineuse, transdermique, locale ou rectale.

Dans les compositions pharmaceutiques de la présente invention pour l'administration orale, sublinguale, parentérale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules, et les solutions ou suspensions orales, les formes d'administration sublinguale et buccale, les formes d'administration parentérale, sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs de goût ou des édulcorants.

Pour une administration rectale, on recourt à des suppositoires qui sont préparés avec des liants fondant à la température rectale, par exemple du beurre de cacao ou des polyéthylène glycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules ou de nanocapsules, éventuellement avec un ou plusieurs supports additifs.

Les composés de l'invention peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier pourra se rendre compte lui-même.

La présente invention a également pour objet une composition pharmaceutique comprenant :
(i) au moins un composé de formule (I) tel que défini ci-dessus, et
(ii) au moins un autre principe actif,
en tant que produits de combinaison pour une utilisation simultanée, séparée ou étalée dans le temps.

En effet, il est classique d'utiliser des bi- ou des tri-thérapies pour traiter un cancer. Le principe actif utilisé sera avantageusement un composé anticancéreux.

A titre d'exemples de principes actifs pouvant être associés au composé de formule (I) dans une composition selon l'invention, on cite de façon non limitative la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide et le lanréotide.

La présente invention a également pour objet une composition pharmaceutique comprenant :
(i) au moins un composé de formule (I) tel que défini ci-dessus, et
(ii) au moins un autre principe actif,
en tant que produits de combinaison pour une utilisation simultanée, séparée ou étalée dans le temps,
pour son utilisation en tant que médicament antiprolifératif, notamment destiné au traitement ou à la prévention du cancer, et en particulier du cancer du sein et de la prostate. Encore plus avantageusement, le médicament sera destiné au traitement ou à la prévention du cancer du sein, de préférence hormono-indépendant.

La présente invention a également pour objet l'utilisation d'un composé de formule (1) tel que défini ci-dessus, pour la fabrication d'un médicament destiné à être administré isolément ou en association, de manière simultanée, séparée ou étalée dans le temps avec des rayonnements ionisants ou non ionisants, en vue de traiter ou prévenir une maladie proliférative telle que le cancer, et en particulier un cancer du sein ou de la prostate, et plus avantageusement un cancer du sein, notamment hormono-indépendant.

La présente invention concerne également une méthode de traitement d'une maladie proliférative, telle que le cancer, et en particulier le cancer du sein ou de la prostate, et encore plus avantageusement le cancer du sein, notamment hormono-indépendant, comprenant l'administration d'une quantité suffisante d'un composé de formule (1) tel que défini ci-dessus, isolément ou en association, de manière simultanée, séparée ou étalée dans le temps, avec des rayonnements ionisants ou non ionisants, à un patient en ayant besoin.

Les rayonnements utilisés peuvent être en particulier des rayons X ou gamma, rayonnements couramment utilisés en radiothérapie dans le traitement du cancer.

La présente invention a également pour objet un procédé de préparation d'un composé de formule (1) tel que défini ci-dessus comprenant les étapes suivantes :
(i) couplage de McMurry entre un composé de formule (II) suivante : 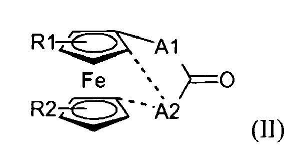 pour laquelle R1, R2, A1 et A2 sont tels que définis précédemment,
   et un composé de formule (III) suivante : 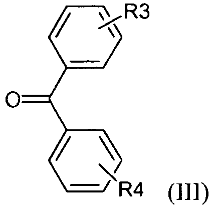 pour laquelle R3 et R4 sont tels que définis précédemment,
   pour former le composé de formule (I),
(ii) récupération du composé de formule (I), obtenu à l'étape (i) précédente.

Le couplage de McMurry peut être éventuellement suivi de réaction de déprotection et/ou de modification et de fonctionnalisation des groupements R3 et R4 par des procédés classiques bien connu de l'homme du métier.

La récupération du composé de l'invention pourra se faire par des méthodes bien connues de l'homme du métier, notamment par filtration ou évaporation du solvant, notamment sous vide. Des étapes de lavage de la phase organique contenant le composé de l'invention et d'extraction pourront être réalisées au préalable.

Le produit obtenu pourra être purifié si nécessaire par des méthodes classiques de purification bien connues de l'homme du métier, telles qu'une recristallisation, une chromatographie sur couche mince préparative, une chromatographie en phase liquide à haute performance (communément appelé HPLC) ou une chromatographie sur colonne de gel de silice. Avantageusement, il s'agira d'une recristallisation, lorsque le produit est cristallin et/ou d'une chromatographie sur colonne de gel de silice.

Le couplage de McMurry est décrit notamment dans les publications suivantes : Nakayama J. et al. Chem. Com. 1986, 12, 974-975 ; Top S. et al. Chem. Eur. J. 2003, 9, 5223-5236 ; Vessieres A. et al. J. Med. Chem. 2005, 48, 3937-3940 ; ou encore Hillard E.A. et al. Dalton Transactions 2007, 43, 5073-5081.

Le couplage de McMurry utilise comme réactif un complexe de titane de faible valence, tel que TiCl₄ ou TiCl₃, en présence d'un agent réducteur, tel que du lithium, du sodium, du magnésium, du zinc, LiAlH₄ ou encore un amalgame Zn-Cu.

De préférence, la réaction de couplage de McMurry sera réalisée en présence de TiCl₄ et de zinc, de préférence sous forme de poudre, et notamment en présence de pyridine.

Le composé de formule (II) pourra être obtenu par des procédés bien connu de l'homme du métier et notamment décrits dans les articles suivants : Turbitt T.D. et al. J. of Organomet. Chem. 1972, 46, 109-117 ; Rosenblum M. et al. J. Am. Chem. Soc. 1963, 85, 316-324 ; Movk W. et al. J. Org. Chem. 1962, 27, 4050-4051 ; Locke A.J. et al. Organometallics 1999, 18, 3750-3759 ; Bickert P. et al. Organometallics 1984, 3, 653-657 ; Kenneth L. et al. J. Am. Chem. Soc. 1962, 84(17), 3263-3269 et Radovan Sebesta et al. Tetrahedron Asymmetry 2007, 18(16), 1893-1898.

Le composé de formule (III) pourra être soit disponible commercialement, soit préparé par des méthodes bien connues de l'homme du métier. La 4,4'-dihydroxybenzophénone est notamment commercialisée par Alfa Aeser.

Les exemples qui suivent vont permettre de mieux comprendre la présente invention sans pour autant en limiter la portée.

### EXEMPLES

Abréviations utilisées :
- RMN: Résonance Magnétique Nucléaire
- IR: Infra Rouge
- SM: Spectrométrie de Masse
- SMHR: Spectrométrie de Masse Haute Résolution

### Exemple 1 : Préparation des composés de l'invention

### 1.1. Préparation de 1,1'-[1-[1,1-bis(4-hydroxyphényl)méthylidène]triméthylène] ferrocène (4)

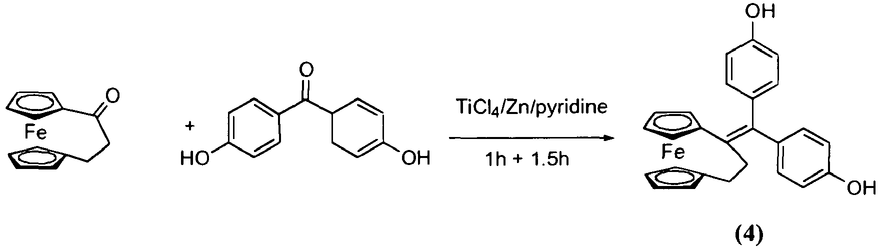

A une suspension de 3,07 g (0,0469 mmol) de poudre de zinc dans 100 cm³ de tétrahydrofurane (THF) anhydre, sont ajoutés 4,51g (2,61 cm³; 0,024 mol) de TiCl₄ sous agitation et à -10°C. Le bain de refroidissement est ensuite retiré et le mélange réactionnel est mis au reflux pendant 60 min. Après refroidissement à température ambiante (t.a.), 3,79g (3,86 cm³; 0,0479 mol) de pyridine anhydre sont ajoutés et le mélange est agité pendant 5 min à t.a.. Une solution de 1,92 g (0,008 mol) de [3]ferrocénophan-1-one (préparé selon Turbitt T.D. et al. J. of Organomet. Chem. 1972, 46, 109-117 ou Rosenblum M. et al. J. Am. Chem. Soc. 1963, 85, 316-324) et de 1,71g (0,008 mol) de 4,4'-dihydroxybenzophénone dans 30 cm³ de THF anhydre est ensuite ajoutée et le mélange réactionnel est mis au reflux pendant 90 min. Après refroidissement à température ambiante, le mélange résultant est hydrolysé avec 100 cm³ d'une solution aqueuse de carbonate de potassium à 8%. Le mélange réactionnel est extrait plusieurs fois avec 100 cm³ d'éther diéthylique. La phase organique est lavée avec de l'eau (2 x 200ml) et de la saumure (200 ml), séchée sur sulfate de magnésium et évaporée jusqu'à siccité. Le produit 1 est isolé par chromatographie flash sur gel de silice (800 ml, éluant n-pentane - éther diéthylique 2 : 1) avec un rendement de 46,50% (pureté 90-95%).

Si nécessaire, le composé **4** ainsi obtenu peut être purifié au moyen d'une seconde chromatographie flash utilisant 60 ml de gel de silice et un mélange n-pentane - éther diéthylique 4 : 1 comme éluant, pour donner une poudre jaune qui est recristallisée dans un mélange acétate d'éthyle (5 ml) - n-pentane (80 ml) à 4 °C. Après 48h, la solution organique est éliminée et les cristaux jaunes obtenus sont lavés deux fois au pentane pour conduire au composé 4 pure avec un rendement de 28,14%.

Selon une seconde variante, le produit brut peut être dissout dans une quantité minimale d'acétone chaude et laissé cristalliser pendant 24h à -20 °C. Le solvant est retiré par décantation et les cristaux obtenus sont lavés deux fois avec 10 ml de n-pentane pour donner 1,10 g du composé **4** pure.
RMN ¹H (300,13 MHz, acétone-d₆): 8,31 (s, 1H, OH); 8,10 (s, 1H, OH); 7,07 (d, *J*=8,40 Hz, 2H, Ar); 6,85 (d, *J*=8,70 Hz, 2H, Ar); 6,84 (d, *J*=8,70 Hz, 2H, Ar); 6,54 (d, *J*=8,70 Hz, 2H, Ar); 4,25 (t, *J*=1,80 Hz, 2H, Cp); 3,98 (m, 4H, Cp); 3,92 (t, J=1,50 Hz, 2H, Cp); 2,70 (m, 2H, CH₂); 2,34 (m, 2H, CH₂)
RMN ¹³C (75,48 MHz, acétone-d₆): 156,95; 156,35; 141,41; 136,36; 132,40; 131,14; 115,68; 114,82; 87,62; 84,89; 70,96; 70,83; 69,14; 68,78; 41,50; 29,14
Analyse: Calculé pourC₂₆H₂₂FeO₂ : C - 73,95 %, H -5,25 % , Trouvé : C - 73,79 %, H - 5,34 %
SM (IC - CH₄): 423,1 (M+H)⁺; 422,1 (M)⁺;
SMHR (IC - CH₄): trouvé: 423,1040 (M+H)⁺, calculé pour C₂₆H₂₃FeO₂ 423,1047 (M+H)⁺

### 1.2. Préparation de 1,1'-{2-[1,1-bis(4-hydroxyphényl)méthylidène]triméthylène] ferrocène (5)

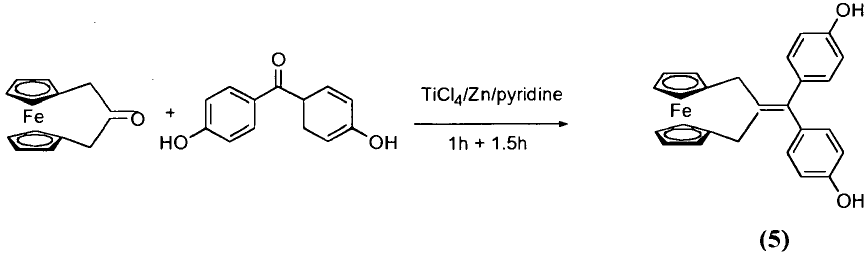

Le composé 5 a été préparé selon le protocole décrit pour le composé 4 à partir de 0,100g (4,167x10⁻⁴ mol) de 1,1'-(2-kétotriméthylène)ferrocène (préparé selon Movk W. et al. J. Org. Chem. 1962, 27, 4050-4051), de 0,0892g (4,167x10⁻⁴ mol) de 4,4'-dihydroxybenzophénone, de 0,235g (0,136 cm³, 1,250x10⁻³ mol) de TiCl₄, de 0,160g (2,443x10⁻³ mol) de poudre de zinc, de 0,197g (0,201 cm³, 2,500x10⁻³ mol) de pyridine anhydre et de 5 + 1,5 cm³ de tétrahydrofurane anhydre.

Le composé **5** a été isolé sous forme d'une poudre jaune par chromatographie flash sur gel de silice (80 ml, éluant n-pentane - éther diéthylique 3 : 4) avec un rendement de 22%.

Le composé ainsi obtenu par chromatographie peut être purifié davantage en le dissolvant dans une quantité minimale d'acétone chaude et en le laissant cristalliser pendant 24h à -20 °C. Le solvant est retiré par décantation et les cristaux obtenus sont lavés deux fois avec 2 ml de n-pentane pour donner 0,045 g du composé 5 pure.
RMN ¹H (300,13 MHz, acétone-d₆): 8,22 (s, 1H, OH); 7,16 (d, J=8,7 Hz, 2H, Ar): 6,80 (d, J=8,7 Hz, 2H, Ar); 4,11 (t, J=1,9 Hz, 2H, Cp); 3,99 (t, J=1,9 Hz, 2H, Cp); 2,81 (s, CH₂);
RMN ¹³C (75,48 MHz, acétone-d₆): 156,66; 156,56; 141,67; 138,59; 135,80; 130,98; 115,73; 115,64; 83,52; 70,28; 69,23:
IR (KBr): 3407; 1609; 1508; 1430; 1220; 1197; 831
SM (IC - CH₄): 423,1 (M+H)⁺; 422,1 (M)⁺;
SMHR (IC - CH₄): trouvé : 423,1034 (M+H)⁺, calculé pour C₂₆H₂₃FeO₂ 423,1047 (M+H)⁺

### 1.3. Préparation de (S)-1,1'-[1-[1,1-bis(4-hydroxyphényl)méthylidène]-3-méthyl-triméthylène]ferrocène (6)

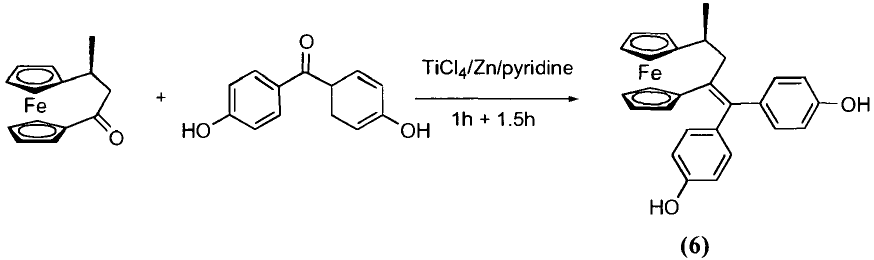

Le composé 6 a été préparé selon le protocole décrit pour le composé 4 à partir de 0,254g (1x10⁻³ mol) de (S)-1,1'(3-méthyl-1-kétotriméthylène)ferrocène (préparé selon Locke A.J. et al. Organometallics 1999, 18, 3750-3759), de 0,214g (1x10⁻³ mol) de 4,4'-dihydroxybenzophénone, de 0,564g (0,326 cm³, 3,000x10⁻³ mol) de TiCl₄, de 0,384g (5,862x10⁻³ mol) de poudre de zinc, de 0,472g (0,482 cm³, 6,000x10⁻³ mol) de pyridine anhydre et de 12 + 4 cm³ de tétrahydrofurane anhydre.

Le composé 6 a été isolé sous forme d'une poudre jaune par chromatographie flash sur gel de silice (80 ml, éluant n-pentane - éther diéthylique 1 : 1) et recristallisation dans un mélange éther diéthylique - pentane avec un rendement de 24,08%.
RMN ¹H (300,13 MHz, acétone-d₆): 8,30 (s, 1H, OH); 8,09 (s, 1H, OH); 7,06 (d, J=8,7 Hz, 2H, Ar); 6,83 (d, J=8,7 Hz, 4H, Ar); 6,53 (d, J=8,7 Hz, 2H, Ar); 4,32 (m, 2H); 4,22 (m, 1H); 4,07 (m, 1H); 4,01 (m, 1H); 3,82 (m, 2H); 3,68 (m, 1H); 2,60 (m, 1H); 1,13 (d, J=6,4 Hz, 3H)
RMN ¹³C (75,48 MHz, acétone-d₆): 156,96; 156,38; 136,04; 135,86; 133,74; 132,43; 131,24; 130,43; 115,61; 114,85; 92,68; 86,90; 71,83; 69,98; 69,80; 69,22; 68,72; 67,51; 66,83; 66,07; 50,23; 36,23; 22,46; 15,57

### 1.4. Préparation de (R)-1,1'-[1-[1,1-bis(4-hydroxyphényl)méthylidène]-3-méthyl-triméthylène]ferrocène (7)

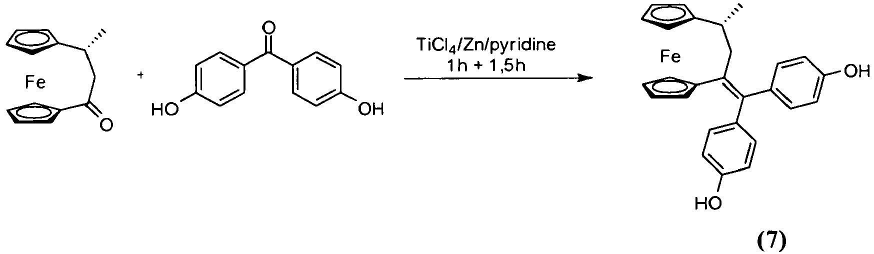

Le composé 7 a été préparé selon le protocole décrit pour le composé 4 à partir de 0,254g (1x10⁻³ mol) de (R)-1,1'(3-méthyl-1-kétotriméthylène)ferrocène (préparé selon Locke A.J. et al. Organometallics 1999, 18, 3750-3759), de 0,214g (1x10⁻³ mol) de 4,4'-dihydroxybenzophénone, de 0,564g (0,326 cm³, 3,000x10⁻³ mol) de TiCl₄, de 0,384g (5,862x10⁻³ mol) de poudre de zinc, de 0,472g (0,482 cm³, 6,000x10⁻³ mol) de pyridine anhydre et de 12 + 4 cm³ de THF anhydre.

Le composé 7 a été isolé sous forme d'une poudre jaune par chromatographie flash sur gel de silice (80 ml, éluant n-pentane - éther diéthylique 1 : 1) et recristallisation dans un mélange éther diéthylique - pentane avec un rendement de 24,08%.
RMN ¹H (300,13 MHz, acétone-d₆): 8,30 (s, 1H, OH); 8,09 (s, 1H, OH); 7,06 (d, J=8,7 Hz, 2H, Ar); 6,83 (d, J=8,7 Hz, 4H, Ar); 6,53 (d, J=8,7 Hz, 2H, Ar); 4,32 (m, 2H); 4,22 (m, 1H); 4,07 (m, 1H); 4,01 (m, 1H); 3,82 (m, 2H); 3,68 (m, 1H); 2,60 (m, 1H); 1,13 (d, J=6,4 Hz, 3H)
RMN ¹³C (75,48 MHz, acétone-d₆): 156,96; 156,38; 136,04; 135,86; 133,74; 132,43; 131,24; 130,43; 115,61; 114,85; 92,68; 86,90; 71,83; 69,98; 69,80; 69,22; 68,72; 67,51; 66,83; 66,07; 50,23; 36,23; 22,46; 15,57

### 1.5. Préparation de 1,2-[1-[1,1-bis(4-hydroxyphényl)méthylidène]triméthylène] ferrocène (8)

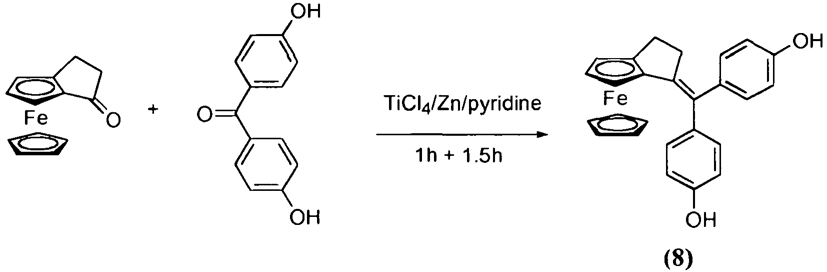

Le composé 8 a été préparé selon le protocole décrit pour le composé 4 à partir de 0,120g (5x10⁻⁴ mol) de 1,2-(1-kétotriméthylène)ferrocène (préparé selon Bickert P. et al. Organometallics 1984, 3, 653-657), de 0,107g (5x10⁻⁴ mol) de 4,4'-dihydroxybenzophénone, de 0,282g (0.163 cm³, 1,500x10⁻³ mol) de TiCl₄, de 0,192g (2,931x10⁻³ mol) de poudre de zinc, de 0,236g (0,241 cm³, 3,000x10⁻³ mol) de pyridine anhydre et de 6 + 1,8 cm³ de THF anhydre.

Le composé 8 a été isolé sous forme d'une poudre orange avec un rendement de 30% par chromatographie flash sur gel de silice (50 ml, éluant n-pentane - éther diéthylique 3 : 4) suivie d'un seconde chromatographie utilisant 80 ml de gel de silice et un mélange n-pentane - éther diéthylique 1 : 1 comme éluant.
RMN ¹H (300,13 MHz, acétone-d₆): 8,27 (s, 1H, OH); 8,24 (s, 1H, OH); 7,15 (d, J=8,7 Hz, 2H, Ar); 7,07 (d, J=8,7 Hz, 2H, Ar); 6,86 (d, J=8,7Hz, 2H, Ar); 6,78 (d, J=8,7Hz, 2H, Ar); 4,14 (m, 1H, Cp); 4,03 (s, 5H, Cp); 3,95 (m, 1H, Cp); 3,58 (m, 1H, CH₂); 3,16 (m, 1 H, Cp); 2,90 (m, 1H, CH₂); 2,70 (m, 1H, CH₂); 2,49 (m, 1H, CH₂)
RMN ¹³C (75,48 MHz, acétone-d₆): 156,92; 156,50; 137,74; 135,60; 131,91; 131,61; 130,82; 130,43; 115,75; 115,49; 98,12; 91,64; 71,44; 70,59; 69,39; 62,92; 62,68; 37,08; 25,53
Analyses : Calculé pour C₂₆H₂₂FeO₂ : C - 73,95 %, H -5,25 % , Trouvé : C - 73,61 %, H - 5,77 %
SM (IE): 422 (M)⁺
SMHR (IE): trouvé : 422,0953 (M)⁺, calculé pour C₂₆H₂₂FeO₂ 422,0969 (M)⁺

### 1.6. Préparation de 1,2-[1-[1,1-bis(4-hydroxyphényl)méthylidène] tétraméthylène]ferrocène (9)

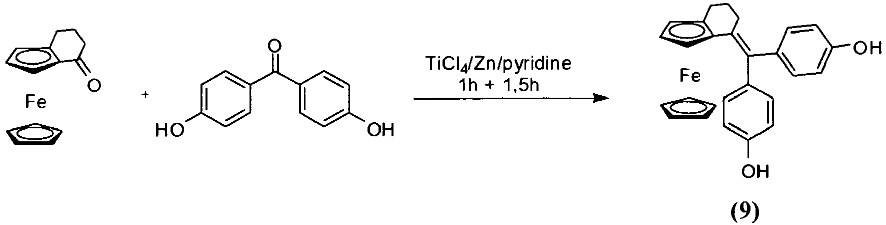

Le composé **9** a été préparé selon le protocole décrit pour le composé **4** à partir de 0,508g (2x10⁻³ mol) de 1,2-(1-kétotétraméthylène)ferrocène (préparé selon Kenneth L. et al. J. Am. Chem. Soc. 1962, 84(17), 3263-3269), de 0,428g (2x10⁻³ mol) de 4,4'-dihydroxybenzophénone, de 1,128g (0,658 cm³, 6,00x10⁻³ mol) de TiCl₄, de 0,784g (11,724x10⁻³ mol) de poudre de zinc, de 0,944g (0,970 cm³, 12,00x10⁻³ mol) de pyridine anhydre et de 28 + 10 cm³ de THF anhydre.

Le composé **9** a été isolé par chromatographie flash sur gel de silice (50 ml, éluant n-pentane - éther diéthylique 2: 1).

Selon une seconde variante, le composé **9** peut être purifié en dissolvant le produit brut dans une quantité minimale d'acétone chaude et en le laissant cristalliser pendant 24h à -20 °C. Le solvant est ensuite retiré par décantation et les cristaux ainsi obtenus sont lavés deux fois avec 2 ml de n-pentane pour donner 0,140 g (16%) du composé **9** pure.
RMN ¹H (700,45 MHz, acétone-d₆): 8,31 (s, OH); 8,35 (s, OH); 7.05 (d, J=8,4 Hz, 4H, Ar); 6,87 (m, 2H, Ar); 6,78 (d, J=8,8 Hz, 2H, Ar); 4,15 (s, 1 H, Cp); 4,02 (s, 5H, Cp); 3,85 (t, J=2,2 Hz, 1H, Cp); 3.15 (m, 1H, Cp); 2,80-2,75 (m, 2H, CH₂); 2,41-2,34 (m, 2H, CH₂); 2,03-1,99 (m, 1H, CH₂); 1,67-1,63 (m, 1H, CH₂)

### 1.7. Préparation de 1,1'-[1-[1,1-bis(4-hydroxyphényl)méthylidène] pentaméthylène]ferrocène (10)

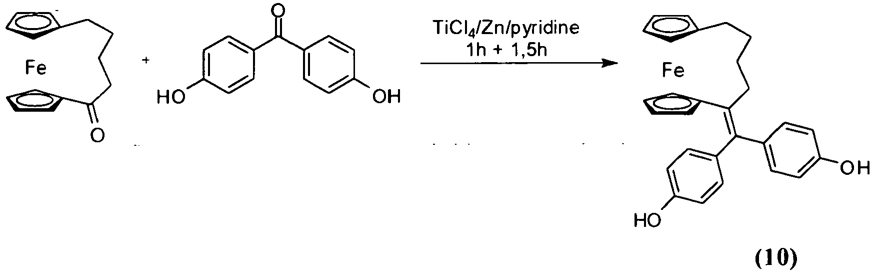

Le composé **10** a été préparé selon le protocole décrit pour le composé **4** à partir de 1,1' -(1-kétopentaméthylène)ferrocène (prepare selon Radovan Sebesta et al. Tetrahedron Asymmetry 2007, 18(16), 1893-1898), de 0,214g (1x10⁻³ mol) de 4,4'-dihydroxybenzophénone, de 0,564g (0,329 cm³, 3,00x10⁻³ mol) de TiCl₄, de 0,392g (5,862x10⁻³ mol) de poudre de zinc, de 0,472g (0,485 cm³, 12,00x10⁻³ mol) de pyridine anhydre et de 14 + 5 cm³ de THF anhydre.

Le composé **10** a été isolé par chromatographie flash sur gel de silice (70 ml, éluant n-pentane - éther diéthylique 2:1).

Selon une seconde variante, le composé **10** peut être purifié en dissolvant le produit brut dans une quantité minimale d'acétone chaude et en le laissant cristalliser pendant 24h à -50 °C. Le solvant est ensuite retiré par décantation et les cristaux ainsi obtenus sont lavés deux fois avec 2 ml de n-pentane pour donner 0,050 g (11%) du composé **10** pure.
RMN ¹H (700,45 MHz, acétone-d₆): 8,21 (s, 1H, OH); 8,12 (s, 1H, OH); 7,06 (d, J=8,8 Hz, 2H, Ar); 6,82 (d, J=8,8 Hz, 2H, Ar); 6,81(d, J=8,8 Hz, 2H, Ar); 6,66 (d, J=8,8 Hz, 2H, Ar); 4,06 (t, J=1,8 Hz, 2H, Cp); 4,04 (t, J=1,8 Hz, 2H, Cp); 3,96 (t, J=1,8 Hz, 2H, Cp); 3,82 (t, J=1,8 Hz, 2H, Cp); 2,60 (t, J=6,6 Hz, 2H, CH₂); 2,51 (t, J=6,6 Hz, 2H, CH₂); 2,27 (m, 2H, CH₂); 1,91 (m, 2H, CH₂)
RMN ¹³C (176,15 MHz, acétone-d₆): 139,22; 136,62; 136,45; 133,62; 130,56; 114,82: 88,02; 85,93; 69,97; 68,88; 67,32; 67,03; 31,58; 25,60; 25,17; 23,74

### 1.8. Réparation de 1,1'-[1-[1,1-bis(4-hydroxyphényl)méthylidène]-2-méthyl-triméthylène]ferrocène (11)

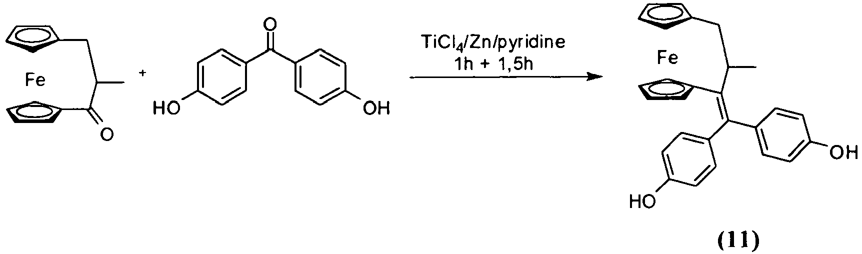

Le composé **11** a été préparé selon le protocole décrit pour le composé **4** à partir de 0,254g (1x10⁻³ mol) de 1,1'(2-méthyl-1-kétotriméthylène)ferrocène (péraparé selon Turbitt T.D. et Watts W.E. J. Organomet. Chem. 1972, 46, 109-117), de 0,214g (1x10⁻³ mol) de 4,4'-dihydroxybenzophénone, de 0,564g (0,326 cm³, 3,000x10⁻³ mol) de TiCl₄, de 0,384g (5,862x10⁻³ mol) de poudre de zinc, de 0,472g (0,482 cm³, 6,000x10⁻³ mol) de pyridine anhydre et de 14 + 5 cm³ de THF anhydre.

Le composé **11** a été isolé sous forme d'une poudre jaune avec un rendement de 5,9% par chromatographie flash sur gel de silice (80 ml, éluant n-pentane - éther diéthylique 3 : 1) suivie d'une seconde chromatographie sue gel de silice (50ml, éluant n-pentane - éther diéthylique 3 : 1).

### 1.8. Mélange des isomers E et Z de 1,1'-{1-[(4-hydroxyphényl) (phényl)méthylidène]triméthylène}ferrocène (12)

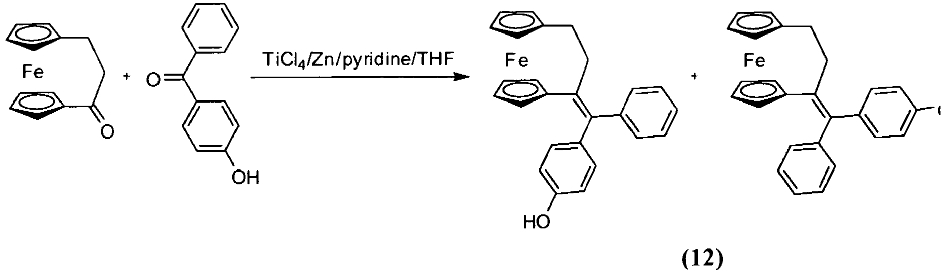

Le composé **12** a été préparé selon le protocole décrit pour le composé **4** à partir de [3]ferrocénophan-1-one (0,192 g, 0,80 mmol), de 4-hydroxybenzophénone (0,158 g, 0,80 mmol), de TiCl₄ (0,451 g, 0,261 cm³, 2,4 mmol), de poudre de zinc (0,307 g, 4,694 mmol), de pyridine anhydre (0,379 g, 0,386 cm³, 4,791 mmol) et de 10 + 3 cm³ de THF anhydre.

Le composé **12** a été isolé sous forme d'une poucre jaune par chromatographie flash sur gel de silice (80 ml, éluant n-pentane - éther diéthylique 35 : 15) suivie d'une seconde chromatographie sue gel de silice (100ml, éluant n-pentane - éther diéthylique 3 : 1).

Le composé 12 a finalement été recristallisé dans un mélange de 10 mL d'acétate d'éthyle et de 80 mL de n-pentane pour donner un mélange des deux isomères dans un ration 1 :4,4.
RMN ¹H (300,13 MHz, acetone-d₆): 8,32 and 8,13 (s, OH); 7,39-7,29 (m, 2H, Ph); 7,28-7,23 (m, 2H, Ph); 7,10-7,01 (m, 1H, Ph); 6,87-6,82 (m, 2H, Ar); 6,57-6,52 (m, 2H, Ar); 4,25 (t, J=1,9 Hz, 2H, Cp); 4,01 and 3,95 (t, J=1,9 Hz, 2H, Cp); 3,98 (t. J=1,9 Hz, 2H, Cp); 3,93 (t, J=1,9 Hz, 2H, Cp); 2,76-2,72 and 2,67-2,64 (m, 2H, CH₂); 2,38-2,31 (m, 2H, CH₂)
RMN ¹³C (75,48 MHz, acetone-d₆): 144,98; 141,57; 135,39; 134,31; 132,35; 131,19; 131,17; 129,98; 128,94; 128,00; 127,35; 114,96; 87,49; 84,52; 71,01; 70,94; 70,92; 69,26; 69,23; 68,87; 68,85; 41,29; 29,16
1R (KBr, cm⁻¹): 2922,9; 2842,1; 1608,3; 1509,2; 1439,2; 1425,9; 1260,9; 1229,2; 1168,9; 1029,3; 830,8; 813,3; 699,6
Analyses : Calculé pour C₂₆H₂₂FeO : C - 76,86 %, H -5,46 % , trouvé: C - 75,71 %, H - 5,55%

### Exemple 2 : Etude des effets antiprolifératifs des composés de l'invention sur des lignées de cancer du sein et de la prostate

Les effets antiprolifératifs des composés de l'invention ont été étudiés sur des cellules MDA-MB-231, qui sont des cellules de cancer du sein hormono-indépendantes, sur des cellules PC-3, qui sont des cellules du cancer de la prostate hormono-indépendantes et sur des cellules MCF-7, qui sont des cellules de cancer du sein hormono-dépendantes.

Le protocole expérimental utilisé pour effectuer cette étude est décrit dans Hillard E. A. et al. Chem. Med. Chem. 2006, 1, 551-559.

Les résultats obtenus sur les lignées MDA-MB-231 et PC-3 sont indiqués dans le Tableau 1 suivant montrant ainsi les propriétés antiprolifératives des composés de l'invention.

**Tableau 1**

| Composés testés | Effet antiprolifératif sur différentes lignées de cellules (IC₅₀ en µM) | |
|---|---|---|
| | MDA-MB-231 | PC-3 |
| 4 | 0,08 | 0,94 |
| 5 | 0,96 | 1,08 |
| 6 | 2,7 | - |
| 7 | 0,78 | - |
| 8 | 2,7 | - |
| 9 | 0,5 | - |
| 11 | 0,63 | - |
| 12 | 0,47 | - |

| | | |
|---|---|---|
| - signifie que le composé de l'invention n'a pas été testé sur la lignée de cellules | | |

Une étude comparative sur la lignée MDA-MB-231 entre les composés de l'invention et les composés ouverts a permis de montrer que les composés de l'invention présentent une meilleure activité cytotoxique que les composés ouverts, comme le montre le Tableau 2 suivant avec les composés a et 12.

**Tableau 2**

| Composé de l'invention | | Composé ouvert |
|---|---|---|
| **4** | 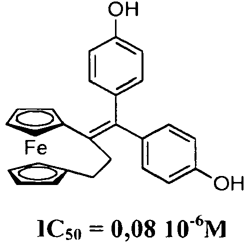 | 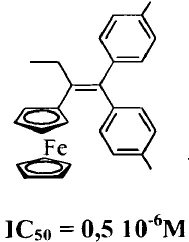 |
| **12** | 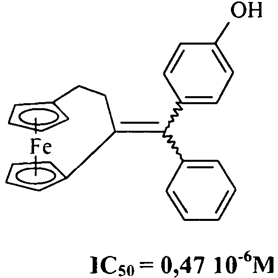 | 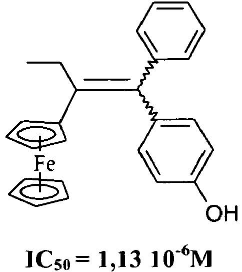 |

Par ailleurs, sur la lignée de cellules MCF-7 (cellules contenant le récepteur alpha des oestrogènes ERα), on note pour **4** un double effet. A très faible concentration, la nature oestrogénique de **4,** mesurée sans rouge phénol ce qui est favorable à l'expression d'un effet oestrogénique, se révèle (effet prolifératif de 162% à 10⁻⁸M par rapport au contrôle, contre 264% pour l'oestradiol). L'effet proprement cytotoxique se révèle à plus fortes concentrations où l'on observe par conséquent la résultante des deux composantes (effet oestrogénique et activité cytotoxique). Par conséquent, il est difficile d'obtenir des valeurs d'IC₅₀ avec une grande précision pour la lignée MCF-7. On obtient cependant les valeurs suivantes : environ 4.10⁻⁶M pour le composé **4** et environ 10⁻⁶M pour le composé **5.**

Ceci implique que la molécule 4, si l'on souhaite l'utiliser en l'état, devra être réservée à des cancers ne contenant pas ERα. Pour les cancers contenant le récepteur ERα, il sera alors nécessaire de masquer l'effet oestrogénique du composé 4, notamment en remplaçant un des phénols par un phényle substitué par la chaîne -O(CH₂)ₓ-N(CH₃)₂ (avec x=2 à 8 par exemple).

De plus la présence des deux phénols sur la molécule 4 permet de pouvoir facilement modifier la lipophilie de la molécule en substituant un ou les deux groupements OH, permettant ainsi de pouvoir améliorer sa biodisponibilité.

## Revendications

1. Composé de formule (I) suivante : 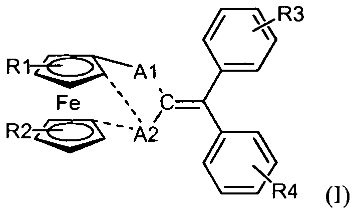 ou un sel pharmaceutiquement acceptable de celui-ci, un isomère ou un mélanges d'isomères en toutes proportions, en particulier un mélange d'énantiomères, et notamment un mélange racémique, ou un dérivé hydrosoluble,
pour lequel :
- A1 et A2 représentent, indépendamment l'un de l'autre, soit une liaison entre le cyclopentadiényle et le carbone de la double liaison, soit une chaîne alkyle linéaire comprenant respectivement n1 et n2 atomes de carbone, éventuellement substituée par un ou plusieurs groupement(s) choisi(s) parmi un atome d'halogène ; un groupe phényle éventuellement substitué par un groupe OH ; un groupe (C₁-C₆)alkyle éventuellement substitué par un ou plusieurs atome(s) d'halogène ; et un groupe (C₃-C₆)cycloalkyle éventuellement substitué par un ou plusieurs atome(s) d'halogène,
avec n1 et n2 représentant, indépendamment l'un de l'autre, un nombre entier compris entre 1 et 4,
à la condition que A1 et A2 ne représentent pas simultanément une liaison et que la chaîne A1-C-A2 comporte au moins 3 atomes de carbone, et de préférence 3, 4 ou 5 atomes de carbone,
- est absent ou représente une liaison, à la condition que A2 soit lié à un et un seul cylopentadiényle,
- R1 et R2 représentent chacun un atome d'hydrogène ou forment ensemble une chaîne alkyle linéaire reliant les deux cyclopentadiényles et comportant 3 à 5 atomes de carbone, cette chaîne étant éventuellement substituée par un ou plusieurs groupement(s) choisi(s) parmi un atome d'halogène ; un groupe phényle éventuellement substitué par un groupe OH ; un groupe (C₁-C₆)alkyle éventuellement substitué par un ou plusieurs atome(s) d'halogène ; et un groupe (C₃-C₆)cycloalkyle éventuellement substitué par un ou plusieurs atome(s) d'halogène, et
- R3 et R4 représentent, indépendamment l'un de l'autre, un atome d'hydrogène ou un groupement CF₃, CN, OR⁵ ou NR⁶R⁷,
avec:
- R⁵ représentant un atome d'hydrogène ou un groupement -CO-(C₁-C₂₀)alkyle ou -(CH₂)ₘNR⁸R⁹,
- R⁶, R⁷, R⁸ et R⁹ représentant, indépendamment les uns des autres, un atome d'hydrogène ou un groupe (C₁-C₆)alkyle ou acyle, et
- m représentant un nombre entier compris entre 2 et 8.

2. Composé selon la revendication 1 **caractérisé en ce que** R3 et/ou R4, et de préférence R3 et R4, représentent, indépendamment l'un de l'autre, un groupement OR⁵, R⁵ étant tel que défini à la revendication 1, et représentant de préférence un atome d'hydrogène.

3. Composé selon l'une quelconque des revendications 1 et 2, **caractérisé en ce que** R3 et/ou R4, et de préférence R3 et R4, sont situés en position *para* sur le noyau phényle.

4. Composé selon l'une quelconque des revendications 1 à 3, **caractérisé en ce que** R1 et R2 représentent chacun un atome d'hydrogène.

5. Composé selon l'une quelconque des revendications 1 à 4, **caractérisé en ce que** A1 ou A2 représente une liaison.

6. Composé selon l'une quelconque des revendications 1 à 5, **caractérisé en ce qu'**il est choisi parmi les composés de formules 4 à 11 suivants :
| | | | |
|---|---|---|---|
| 4 | 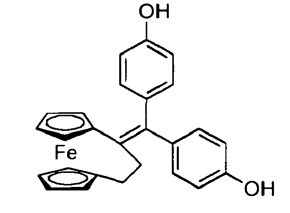 | 5 | 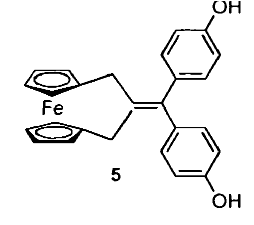 |
| 6 | 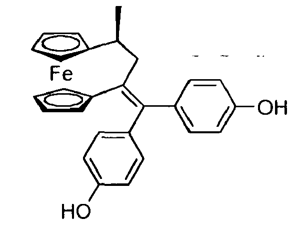 | 7 | 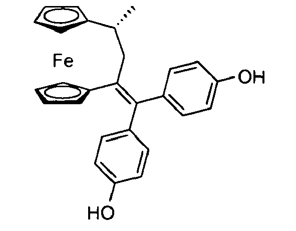 |
| 8 | 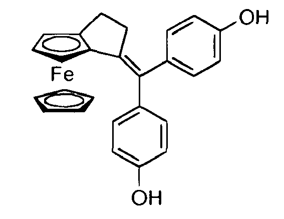 | 9 | 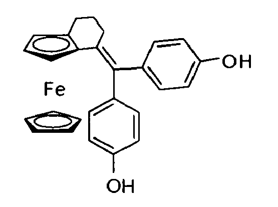 |
| 10 | 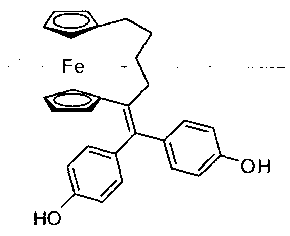 | 11 | 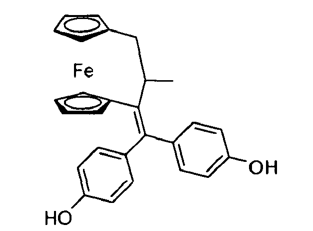 |
| 12 | 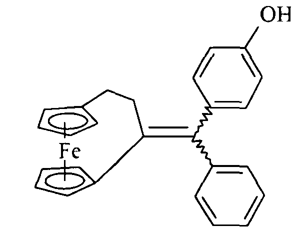 | | |

7. Composé selon l'une quelconque des revendications 1 à 6 pour son utilisation en tant que médicament.

8. Composé selon la revendication 7, pour son utilisation en tant que médicament antiprolifératif, de préférence destiné au traitement ou à la prévention du cancer, et en particulier du cancer du sein ou de la prostate.

9. Composition pharmaceutique comprenant au moins un composé selon l'une quelconque des revendications 1 à 6, en association avec un véhicule pharmaceutiquement acceptable.

10. Composition pharmaceutique selon la revendication 9, **caractérisée en ce qu'**elle comprend au moins un autre principe actif, avantageusement choisi parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide et le lanréotide.

11. Composition pharmaceutique comprenant :
(i) au moins un composé selon l'une quelconque des revendications 1 à 6,
(ii) au moins un autre principe actif,
en tant que produits de combinaison pour une utilisation simultanée, séparée ou étalée dans le temps.

12. Composition selon la revendication 11, **caractérisée en ce que** le(s) principe(s) actif(s) est(sont) choisi(s) parmi la 6-mercaptopurine, la fludarabine, la cladribine, la pentostatine, la cytarabine, le 5-fluorouracile, la gemcitabine, le méthotrexate, le raltitrexed, l'irinotécan, le topotécan, l'étoposide, la daunorubicine, la doxorubicine, l'épirubicine, l'idarubicine, la pirarubicine, la mitoxantrone, la chlorméthine, la cyclophosphamide, l'ifosfamide, le melphalan, le chlorambucil, le busulfan, la carmustine, la fotémustine, la streptozocine, le carboplatine, le cisplatine, l'oxaliplatine, la procarbazine, la dacarbazine, la bléomycine, la vinblastine, la vincristine, la vindésine, la vinorelbine, le paclitaxel, le docétaxel, la L-asparaginase, la flutamide, la nilutamide, la bicalutamide, l'acétate de cyprotérone, la triptoréline, la leuproréline, la goséréline, la buséréline, le formestane, l'aminoglutéthimide, l'anastrazole, le létrozole, le tamoxifène, l'octréotide et le lanréotide.

13. Composition selon l'une quelconque des revendications 11 et 12, pour son utilisation en tant que médicament antiprolifératif, de préférence destiné au traitement ou à la prévention du cancer, et en particulier du cancer du sein ou de la prostate.

14. Utilisation d'un composé selon l'une quelconque des revendications 1 à 6, pour la fabrication d'un médicament destiné à être administré isolément ou en association, de manière simultanée, séparée ou étalée dans le temps avec des rayonnements ionisants ou non ionisants, en vue de traiter ou prévenir un cancer, et en particulier un cancer du sein ou de la prostate.

15. Procédé de préparation d'un composé de formule (1) selon l'une quelconque des revendications 1 à 6 comprenant les étapes suivantes :
(i) couplage de McMurry entre un composé de formule (II) suivante : 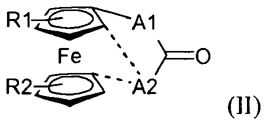 pour laquelle R1, R2, A1 et A2 sont tels que définis à la revendication 1,
et un composé de formule (III) suivante : 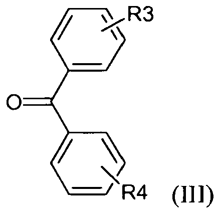 pour laquelle R3 et R4 sont tels que définis à la revendication 1.
pour former le composé de formule (I),
(ii) récupération du composé de formule (I), obtenu à l'étape (i) précédente.

## Patentansprüche

1. Verbindung der folgenden Formel (I): 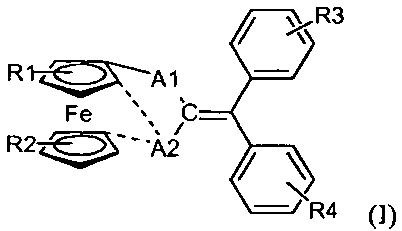 oder pharmazeutisch unbedenkliches Salz davon, Isomer oder Mischung von Isomeren in jeglichen Anteilen, insbesondere Mischung von Enantiomeren und insbesondere racemische Mischung oder wasserlösliches Derivat,
in der:
- A1 und A2 unabhängig voneinander entweder für eine Bindung zwischen dem Cyclopentadienyl und dem Kohlenstoff der Doppelbindung oder eine lineare Alkylkette stehen, die n1 bzw. n2 Kohlenstoffatome umfasst und gegebenenfalls durch eine oder mehrere Gruppen substituiert ist, die aus einem Halogenatom; einer gegebenenfalls durch eine OH-Gruppe substituierten Phenylgruppe; einer gegebenenfalls durch ein oder mehrere Halogenatome substituierten (C₁-C₆)-Alkylgruppe und einer gegebenenfalls durch ein oder mehrere Halogenatome substituierten (C₃-C₆)-Cycloalkylgruppe ausgewählt sind,
wobei n1 und n2 unabhängig voneinander für eine ganze Zahl zwischen 1 und 4 stehen,
unter der Bedingung, dass A1 und A2 nicht gleichzeitig für eine Bindung stehen und dass die Kette A1-C-A2 mindestens 3 Kohlenstoffatome und vorzugsweise 3, 4 oder 5 Kohlenstoffatome umfasst,
- fehlt oder für eine Bindung steht, unter der Bedingung, dass A2 an ein einziges Cyclopentadienyl gebunden ist,
- R1 und R2 jeweils für ein Wasserstoffatom stehen oder zusammen eine lineare Alkylkette bilden, die die zwei Cyclopentadienyle verbindet und 3 bis 5 Kohlenstoffatome umfasst, wobei diese Kette gegebenenfalls durch ein oder mehrere Gruppen substituiert ist, die aus einem Halogenatom; einer gegebenenfalls durch eine OH-Gruppe substituierten Phenylgruppe; einer gegebenenfalls durch ein oder mehrere Halogenatome substituierten (C₁-C₆)-Alkylgruppe und einer gegebenenfalls durch ein oder mehrere Halogenatome substituierten (C₃-C₆)-Cycloalkylgruppe ausgewählt sind, und
- R3 und R4 unabhängig voneinander für ein Wasserstoffatom oder eine CF₃-, CN-, OR⁵- oder NR⁶R⁷-Gruppe stehen,
wobei:
- R⁵ für ein Wasserstoffatom oder eine -CO-(C₁-C₂₀)-Alkyl- oder-(CH₂)ₘNR⁸R⁹-Gruppe steht,
- R⁶, R⁷, R⁸ und R⁹ unabhängig voneinander für ein Wasserstoffatom oder eine (C₁-C₆)-Alkyl- oder -Acylgruppe stehen und
- m für eine ganze Zahl zwischen 2 und 8 steht.

2. Verbindung nach Anspruch 1, **dadurch gekennzeichnet, dass** R3 und/oder R4 und vorzugsweise R3 und R4 unabhängig voneinander für eine OR⁵-Gruppe stehen, wobei R⁵ wie in Anspruch 1 definiert ist, und vorzugsweise für ein Wasserstoffatom stehen.

3. Verbindung nach einem der Ansprüche 1 und 2, **dadurch gekennzeichnet, dass** R3 und/oder R4 und vorzugsweise R3 und R4 sich in *para*-Stellung auf dem Phenylkern befinden.

4. Verbindung nach einem der Ansprüche 1 bis 3, **dadurch gekennzeichnet, dass** R1 und R2 jeweils für ein Wasserstoffatom stehen.

5. Verbindung nach einem der Ansprüche 1 bis 4, **dadurch gekennzeichnet, dass** A1 oder A2 für eine Bindung steht.

6. Verbindung nach einem der Ansprüche 1 bis 5, **dadurch gekennzeichnet, dass** sie aus den Verbindungen der folgenden Formeln 4 bis 11 ausgewählt ist:
| | | | |
|---|---|---|---|
| 4 | 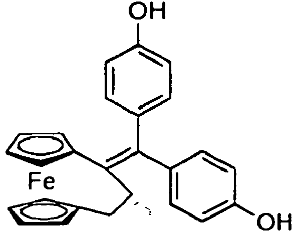 | 5 | 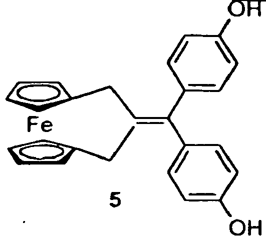 |
| 6 | 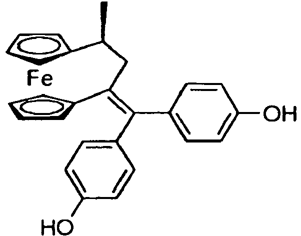 | 7 | 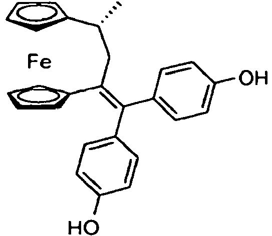 |
| 8 | 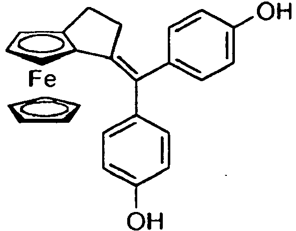 | 9 | 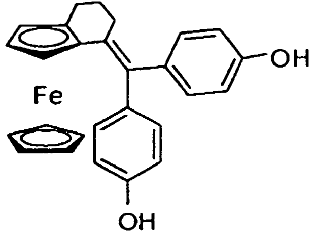 |
| 10 | 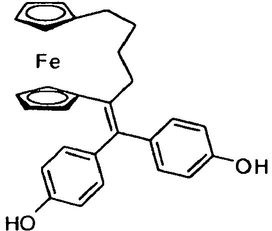 | 11 | 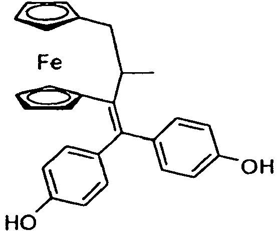 |
| 12 | 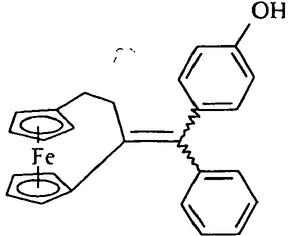 | | |

7. Verbindung nach einem der Ansprüche 1 bis 6 zu deren Verwendung als Arzneimittel.

8. Verbindung nach Anspruch 7 zu deren Verwendung als antiproliferatives Arzneimittel, das vorzugsweise zur Behandlung oder Prävention einer Krebserkrankung und insbesondere einer Brust- oder Prostatakrebserkrankung vorgesehen ist.

9. Pharmazeutische Zusammensetzung, die mindestens eine Verbindung nach einem der Ansprüche 1 bis 6 in Verbindung mit einem pharmazeutisch unbedenklichen Trägerstoff umfasst.

10. Pharmazeutische Zusammensetzung nach Anspruch 9, **dadurch gekennzeichnet, dass** sie mindestens einen weiteren Wirkstoff umfasst, der vorteilhaft aus 6-Mercaptopurin, Fludarabin, Cladribin, Pentostatin, Cytarabin, 5-Fluoruracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid und Lanreotid ausgewählt ist.

11. Pharmazeutische Zusammensetzung, die Folgendes umfasst:
(i) mindestens eine Verbindung nach einem der Ansprüche 1 bis 6,
(ii) mindestens einen weiteren Wirkstoff, ,
als Kombinationsprodukte für eine gleichzeitige, separate oder zeitlich verteilte Verwendung.

12. Zusammensetzung nach Anspruch 11, **dadurch gekennzeichnet, dass** der Wirkstoff bzw. die Wirkstoffe aus 6-Mercaptopurin, Fludarabin, Cladribin, Pentostatin, Cytarabin, 5-Fluoruracil, Gemcitabin, Methotrexat, Raltitrexed, Irinotecan, Topotecan, Etoposid, Daunorubicin, Doxorubicin, Epirubicin, Idarubicin, Pirarubicin, Mitoxantron, Chlormethin, Cyclophosphamid, Ifosfamid, Melphalan, Chlorambucil, Busulfan, Carmustin, Fotemustin, Streptozocin, Carboplatin, Cisplatin, Oxaliplatin, Procarbazin, Dacarbazin, Bleomycin, Vinblastin, Vincristin, Vindesin, Vinorelbin, Paclitaxel, Docetaxel, L-Asparaginase, Flutamid, Nilutamid, Bicalutamid, Cyproteronacetat, Triptorelin, Leuprorelin, Goserelin, Buserelin, Formestan, Aminoglutethimid, Anastrazol, Letrozol, Tamoxifen, Octreotid und Lanreotid ausgewählt ist bzw. sind.

13. Zusammensetzung nach einem der Ansprüche 11 und 12 zu deren Verwendung als antiproliferatives Arzneimittel, das vorzugsweise zur Behandlung oder Prävention einer Krebserkrankung und insbesondere einer Brust- oder Prostatakrebserkrankung vorgesehen ist.

14. Verwendung einer Verbindung nach einem der Ansprüche 1 bis 6 zur Herstellung eines Arzneimittels, das einzeln oder in Verbindung mit ionisierenden oder nicht-ionisierenden Strahlen gleichzeitig, separat oder zeitlich verteilt zwecks Behandlung oder Prävention einer Krebserkrankung und insbesondere einer Brust- oder Prostatakrebserkrankung verabreicht werden soll.

15. Verfahren zur Herstellung einer Verbindung der Formel (I) nach einem der Ansprüche 1 bis 6, wobei das Verfahren die folgenden Schritte umfasst:
(i) Mc-Murry-Kupplung zwischen einer Verbindung der folgenden Formel (II): 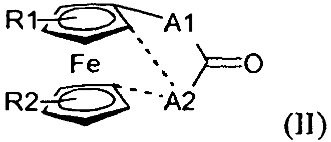 in der R1, R2, A1 und A2 wie in Anspruch 1 definiert sind,
und einer Verbindung der folgenden Formel (III): 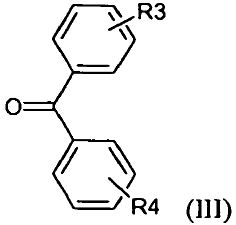 in der R3 und R4 wie in Anspruch 1 definiert sind,
um die Verbindung der Formel (I) zu bilden,
(ii) Rückgewinnung der im vorherigen Schritt (i) erhaltenen Verbindung der Formel (I).

## Claims

1. A compound of the following formula (I): 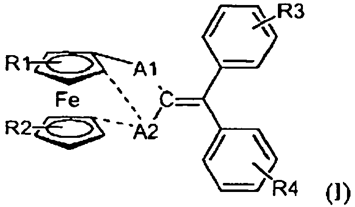 or a pharmaceutically acceptable salt thereof, an isomer or a mixture of isomers in any ratio, in particular a mixture of enantiomers, and more particularly a racemic mixture, or a water-soluble derivative,
wherein:
- A1 and A2, independently from each other, are either a bond between the cyclopentadienyl and the carbon of the double bond, or a linear alkyl chain comprising n1 and n2 carbon atoms, respectively, optionally substituted with one or more group(s) selected from halogen; phenyl optionally substituted with OH; (C₁-C₆)alkyl optionally substituted with one or more halogen (s) ; and (C₃-C₆) cycloalkyl optionally substituted with one or more halogen(s),
wherein n1 and n2 are, independently from each other, an integer comprised between 1 and 4,
with the proviso that A1 and A2 are not simultaneously a bond and that the A1-C-A2 chain contains at least 3 carbon atoms, and preferably 3, 4 or 5 carbon atoms,
- is absent or represents a bond, with the proviso that A2 is linked to one and only one cyclopentadienyl group,
- R1 and R2 each represent hydrogen or together form a linear alkyl chain connecting the two cyclopentadienyl groups and having 3 to 5 carbon atoms, wherein said chain is optionally substituted with one or more group(s) selected from halogen; phenyl optionally substituted with OH; (C₁-C₆)alkyl optionally substituted with one or more halogen(s); and (C₃-C₆)cycloalkyl optionally substituted with one or more halogen(s), and
- R3 and R4, independently from each other, are hydrogen or a CF₃, CN, OR⁵ or NR⁶R⁷ group,
wherein:
- R⁵ is hydrogen or a -CO-(C₁-C₂₀) alkyl or-(CH₂) ₘNR⁸R⁹ group,
- R⁶, R⁷, R⁸ and R⁹ are, independently from one another, hydrogen or a (C₁-C₆)alkyl or acyl group; and
- m is an integer comprised between 2 and 8.

2. The compound according to claim 1 **characterized in that** R3 and/or R4, and preferably R3 and R4, independently from each other, are OR⁵, wherein R⁵ is as defined in claim 1, and is preferably hydrogen.

3. The compound according to either one of claims 1 and 2, **characterized in that** R3 and/or R4, and preferably R3 and R4, are located in the para-position on the phenyl ring.

4. The compound according to any one of claims 1 to 3, **characterized in that** R1 and R2 each represent hydrogen.

5. The compound according to any one of claims 1 to 4, **characterized in that** A1 or A2 represents a bond.

6. The compound according to any one of claims 1 to 5, **characterized in that** it is selected from the compounds of the following formulas 4 to 11:
| | | | |
|---|---|---|---|
| **4** | 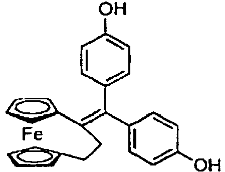 | **5** | 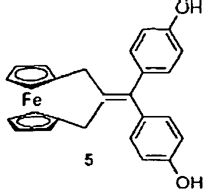 |
| **6** | 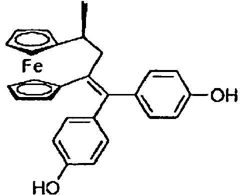 | **7** | 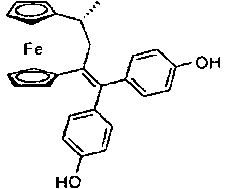 |
| **8** | 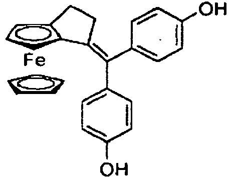 | **9** | 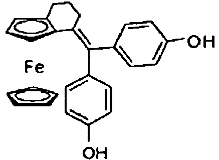 |
| **10** | 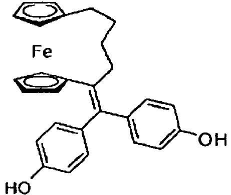 | **11** | 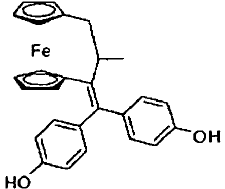 |
| **12** | 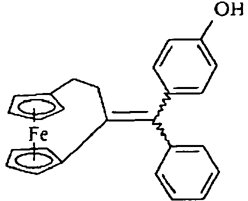 | | |

7. A compound according to any one of claims 1 to 6 for use as a medicine.

8. The compound according to claim 7, for use as an antiproliferative drug, preferably for the treatment or prevention of cancer, and in particular breast or prostate cancer.

9. A pharmaceutical composition comprising at least one compound according to any one of claims 1 to 6, in combination with a pharmaceutically acceptable vehicle.

10. The pharmaceutical composition according to claim 9, **characterized in that** it comprises at least one additional active ingredient, advantageously selected from 6-mercaptopurin, fludarabin, cladribin, pentostatin, cytarabin, 5-fluorouracil, gemcitabin, methotrexate, raltitrexed, irinotecan, topotecan, etoposide, daunorubicin, doxorubicin, epirubicin, idarubicin, pirarubicin, mitoxantrone, chlormethin, cyclophosphamide, ifosfamide, melphalan, chlorambucil, busulfan, carmustin, fotemustin, streptozocin, carboplatin, cisplatin, oxaliplatin, procarbazin, dacarbazin, bleomycin, vinblastin, vincristin, vindesin, vinorelbin, paclitaxel, docetaxel, L-asparaginase, flutamide, nilutamide, bicalutamide, cyproterone acetate, triptorelin, leuprorelin, goserelin, buserelin, formestane, aminoglutethimide, anastrazole, letrozole, tamoxifen, octreotide and lanreotide.

11. A pharmaceutical composition comprising:
(i) at least one compound according to any one of claims 1 to 6,
(ii) at least one additional active ingredient,
as combination products for use simultaneously, separately or sequentially.

12. The composition according to claim 11, **characterized in that** the active ingredient(s) is (are) selected from 6-mercaptopurin, fludarabin, cladribin, pentostatin, cytarabin, 5-fluorouracil, gemcitabin, methotrexate, raltitrexed, irinotecan, topotecan, etoposide, daunorubicin, doxorubicin, epirubicin, idarubicin, pirarubicin, mitoxantrone, chlormethin, cyclophosphamide, ifosfamide, melphalan, chlorambucil, busulfan, carmustin, fotemustin, streptozocin, carboplatin, cisplatin, oxaliplatin, procarbazin, dacarbazin, bleomycin, vinblastin, vincristin, vindesin, vinorelbin, paclitaxel, docetaxel, L-asparaginase, flutamide, nilutamide, bicalutamide, cyproterone acetate, triptorelin, leuprorelin, goserelin, buserelin, formestane, aminoglutethimide, anastrazole, letrozole, tamoxifen, octreotide and lanreotide.

13. The composition according to any one of claims 11 and 12, for use as an antiproliferative drug, preferably for the treatment or prevention of cancer, and in particular breast or prostate cancer.

14. Use of a compound according to any one of claims 1 to 6, for the manufacture of a medicine to be administered alone or in combination, simultaneously, separately or sequentially, with ionizing or non-ionizing radiations, for the treatment or prevention of cancer, and in particular breast or prostate cancer.

15. A process for preparing a compound of the formula (I) according to any one of claims 1 to 6 comprising the following steps:
(i) McMurry coupling of a compound of the following formula (II): 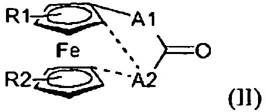 wherein R1, R2, A1 and A2 are as defined in claim 1,
with a compound of the following formula (III): 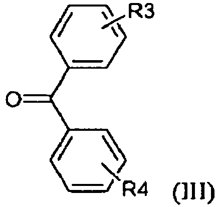 wherein R3 and R4 are as defined in claim 1,
to form the compound of the formula (I),
(ii) recovering the compound of the formula (I), obtained in step (i) above.
